(19) Europäisches Patentamt
European Patent Office
Office européen des brevets

(11) EP 4 782 463 A1

(12) **EUROPEAN PATENT APPLICATION**

(43) Date of publication:
**29.07.2026 Bulletin 2026/31**

(21) Application number: **26170116.3**

(22) Date of filing: **01.04.2026**

(51) International Patent Classification (IPC):
***C07K 14/71*** *(2006.01)* ***A61K 38/00*** *(2006.01)*
***A61P 9/12*** *(2006.01)* ***C12N 5/071*** *(2010.01)*

(52) Cooperative Patent Classification (CPC):
**C07K 14/71; A61K 38/179; A61P 9/12;**
C07K 2319/30; C07K 2319/32

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **30.09.2025 CN 202511424337**

(71) Applicant: **QILU PHARMACEUTICAL CO., LTD.**
**Jinan, Shandong 250100 (CN)**

(72) Inventors:
- **AN, Zhenming**
  **Jinan, Shandong, 250100 (CN)**
- **SUN, Lixia**
  **Jinan, Shandong, 250100 (CN)**
- **WANG, Guijiang**
  **Jinan, Shandong, 250100 (CN)**
- **FAN, Jianmin**
  **Jinan, Shandong, 250100 (CN)**
- **LI, Tao**
  **Jinan, Shandong, 250100 (CN)**
- **LI, Daoyuan**
  **Jinan, Shandong, 250100 (CN)**
- **ZHANG, Jianjun**
  **Jinan, Shandong, 250100 (CN)**
- **XUE, Jiugang**
  **Jinan, Shandong, 250100 (CN)**
- **XIN, Fanghua**
  **Jinan, Shandong, 250100 (CN)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

# (54) A COMPOSITION COMPRISING SOTATERCEPT AND VARIANTS THEREOF, AND RELATED METHODS AND USES

(57) The present application provides a composition comprising Sotatercept and a variant thereof, as well as related methods and uses, wherein the variant has a peptide bond cleavage occurred between arginine at position 4 and serine at position 5 (Arg(4)-Ser(5)) in the amino acid sequence (SEQ ID NO:2) of one or two monomeric peptide chains of sotatercept, and as calculated by a peptide mass fingerprinting analysis, the content of the variant is less than or equal to about 10%. The present application also provides a pharmaceutical formulation comprising the above composition and a method of making the same. The present application further provides a method for detecting the above variant and use of the variant in the quality inspection or quality control of a sotatercept-containing product.

EP 4 782 463 A1

## Description

### Cross-Reference to Related Application

The present application claims priority to Chinese Patent Application No. 202511424337.X, filed on September 30, 2025, the entire contents of which are incorporated herein by reference for all purposes.

### Technical Field

The present application relates to the field of biopharmaceuticals. In particular, the present application provides a composition comprising sotatercept and variants thereof, as well as related methods and uses.

### Background of the Invention

Skeletal diseases, including osteoporosis and bone fractures, represent a group of pathological conditions, and there are very few pharmaceutical formulations that are effective against these pathological conditions. The treatment focuses on physical and behavioral interventions, including immobilization, exercise, and dietary changes. Methods of controlling osteoclast and osteoblast activity are effective in promoting healing of fractures and other bone lesions as well as treatment of diseases (such as osteoporosis, which is associated with loss of bone mass and bone mineralization).

With regard to osteoporosis, estrogen, calcitonin, osteocalcin, and vitamin K, or high doses of dietary calcium, are all used as an intervention therapy. Other therapies of osteoporosis include bisphosphonates, parathyroid hormones, calcium mimetics, statins, salts of lanthanum and strontium, and sodium fluoride. However, these therapies generally produce adverse side effects.

Pulmonary arterial hypertension (PAH) is a serious and progressive disease characterized by abnormal contraction, remodeling (vascular wall thickening, or abnormal cells proliferation) and occlusion of pulmonary arterioles. This results in increased pulmonary vascular resistance, increased right heart load, and ultimately right heart failure and death. In healthy pulmonary vessels, the BMPR-II signaling pathway, (primarily mediated through Smad1/5/8 phosphorylation) is critical to maintain homeostasis of vascular endothelial cells and smooth muscle cells. It has antiproliferative, pro-apoptotic effects and can inhibit excessive growth and migration of vascular cells. When the BMPR-II signal is attenuated or absent (e.g., gene mutation or functional inhibition), this inhibition will be weakened, which will lead to excessive proliferation and increased survival of pulmonary vascular cells, and promote vascular remodeling and PAH development. A key pathological mechanism is dysfunction of bone morphogenetic protein receptor type II (BMPR-II) signaling pathways. A large number of hereditary and sporadic PAH patients have mutations in the BMPR2 gene or impairment of its downstream signal transduction.

The soluble form of ActRIIA (activin receptor IIA) acts as an inhibitor of activin-ActRIIA signaling and promotes increased bone density, bone growth and bone strength in vivo. However, the vast majority of pharmaceutical formulations that promote bone growth or inhibit bone loss are either as anti-catabolic preparations or as anabolic preparations. Soluble ActRIIA protein shows dual activity with catabolic and anabolic effects. Therefore, antagonists of the activin-ActRIIA signaling pathway can be used to increase bone density and promote bone growth.

On the other hand, given that impaired BMPR-II signaling is one of the core pathological mechanisms of PAH, and excessive activation of ligands such as activin/GDF will further suppress the already compromised BMP signaling, researchers have hypothesized that by capturing these inhibitory ligands, the balance of BMP signaling pathway can be restored, thereby inhibiting the pulmonary vascular remodeling process.

This mechanism of action differs from existing PAH targeting drugs (such as endothelin receptor antagonists, phosphodiesterase-5 inhibitors, prostacyclins, guanylate cyclase stimulators), which act primarily on vasomotor tension (vasodilation). Sotatercept directly targets cell proliferation signaling pathways leading to aberrant remodeling of vascular structures, and has the potential of "disease modification".

Sotatercept is an activin receptor IIA-Fc fusion protein. It functions by targeting and blocking the signaling of the TGF-beta superfamily, such as targeting and blocking activin and growth differentiation factors. In PAH, this signaling pathway is overactive, resulting in abnormal thickening and blockage of vascular wall. Sotatercept aims to restore the balance of cell growth in vascular wall, thereby improving the health status of pulmonary vessels.

In addition, sotatercept can dually regulate bone metabolism. It has the effects of resisting catabolism, inhibiting bone loss and promoting anabolism, accelerating bone growth, and significantly improving bone density, bone strength and fracture healing ability. Sotatercept has a unique mechanism different from that of traditional anti-osteoporosis drugs such as bisphosphonate/PTH, and does not increase muscle mass, and is suitable for osteoporosis and bone repair therapy.

As a therapeutic drug, the safety and efficacy of sotatercept are highly dependent on the ability to maintain stability and efficacy of the drug throughout its entire lifecycle. For this reason, the quality control of its pharmaceutical composition is critical, the core of which is to ensure that the content of the active ingredient is in compliance with the formulation specifications, and that the content of related substances such as variants or impurities is strictly controlled to meet the requirements of pharmaceutical regulations, thereby ultimately guaranteeing the medication safety of patients and the expected therapeutic outcomes.

## Summary of the Invention

In a first aspect, the present application provides a composition comprising sotatercept and a variant thereof, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2, and as calculated by a peptide mass fingerprinting analysis, the content of monomeric peptide chains with the peptide bond cleavage occurredbetween Arg(4)-Ser(5) in the composition is less than or equal to about 10% of the total amount of monomeric peptide chains in the composition, i.e., a cleavage rate of the peptide bond between Arg(4)-Ser(5) is less than or equal to about 10%.

In specific embodiments, the sequence of a truncated monomeric peptide chain resulting from the peptide bond cleavage between Arg(4)-Ser(5) is as set forth in SEQ ID NO:1.

The truncated variant resulting from the peptide bond cleavage between Arg(4) -Ser(5) is also referred to herein as $R^4/S^5$ truncated variant.

In specific embodiments, as calculated by a peptide mass fingerprinting analysis, the above cleavage rate of the peptide bond is less than or equal to about 10%, which is determined by using LC-MS/MS.

In specific embodiments, as calculated by a peptide mass fingerprinting analysis, the content of monomeric peptide chains without the peptide bond cleavage occurred between Arg(4)-Ser(5) is greater than or equal to about 90% of the total amount of monomeric peptide chains in the composition, i.e., the non-cleavage ratio of the peptide bonds between Arg(4) -Ser(5) is greater than or equal to about 90%.

In specific embodiments, the cleavage rate of the peptide bond between Arg(4) -Ser(5) is greater than or equal to about 0.01%, greater than or equal to about 0.05%, greater than or equal to about 0.1%, greater than or equal to about 0.5%, greater than or equal to about 1%, greater than or equal to about 1.5%, or greater than or equal to about 2%.

In specific embodiments, the cleavage rate of the peptide bond between Arg(4) -Ser(5) is less than or equal to about 10%, less than or equal to about 9%, less than or equal to about 8%, less than or equal to about 7%, less than or equal to about 6%, less than or equal to about 5%, less than or equal to about 4%, less than or equal to about 3%, less than or equal to about 2.5%, less than or equal to about 2%, less than or equal to about 1.5%, less than or equal to about 1%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, or less than or equal to about 0.1%. In some embodiments, the cleavage rate of the peptide bond between Arg(4) - Ser(5) can be any interval within the ranges defined by any two of the above-mentioned values or any value within the interval.

In some embodiments, the composition is prepared by a method comprising the steps of:
culturing host cells comprising a nucleic acid molecule encoding an amino acid sequence as set forth in SEQ ID NO:2 or a vector comprising the nucleic acid molecule in a bioreactor, the culture comprising a cell expansion phase and a protein production phase, wherein the culture temperature during the protein production phase is substantially maintained at 29.5°C-37°C.

In some embodiments, the composition is prepared by a method comprising the steps of:
culturing host cells comprising a nucleic acid molecule encoding an amino acid sequence as set forth in SEQ ID NO:2 or a vector comprising the nucleic acid molecule in a bioreactor, wherein the average daily addition concentration of iron ions (e.g., ferric citrate) during the culture period is from 1.5 μmol/L/day to 125 μmol/L/day.

In a second aspect, the present application provides a method for preparing the composition disclosed in the first aspect, the method comprising culturing host cells comprising a nucleic acid molecule encoding an amino acid sequence as set forth in SEQ ID NO:2 or a vector comprising the nucleic acid molecule in a bioreactor, wherein the culturing comprises a cell expansion phase and a protein production phase, and the culture temperature of the protein production phase is substantially maintained at 29.5°C-37°C; and/or the culturing is performed using a culture medium supplemented with iron ions.

In specific embodiments, a sotatercept variant is a truncated variant formed by peptide bond cleavage between Arg(4) -Ser(5) at the N-terminus of the amino acid sequence of at least one monomeric peptide chain of sotatercept, and the amino acid sequence of the monomeric peptide chain formed by peptide bond cleavage between Arg(4) -Ser(5) at the N-terminus is as set forth in SEQ ID NO: 1.

In specific embodiments, the iron ions are derived from one or more of ferric nitrate, ferrous sulfate, ferric citrate, ferric ethylenediaminetetraacetate, and transferrin-bound iron.

In specific embodiments, the iron ions are derived from ferric citrate.

In specific embodiments, the average daily addition concentration of the iron ions (e.g., ferric citrate) during the culture period is from 1.5 μmol/L/day to 125 μmol/L/day.

In specific embodiments, the average daily addition concentration of the iron ions (e.g., ferric citrate) is from 1.50 μmol/L/day to 5.00 μmol/L/day, 5.01 μmol/L/day to 15.00 μmol/L/day, 15.01 μmol/L/day to 30.00 μmol/L/day, 30.01 μmol/L/day to 45.00 μmol/L/day, 45.01 μmol/L/day to 60.00 μmol/L/day, 60.01 μmol/L/day to 75.00 μmol/L/day, 75.01 μmol/L/day to 90.00 μmol/L/day, 90.01 μmol/L/day to 115.00μmol/L/day, or 115.01 μmol/L/day to 125.00 μmol/L/day.

In specific embodiments, the average daily addition concentration of the iron ions (e.g., ferric citrate) is selected from 1.77 μmol/L/day, 7.88 μmol/L/day, 62.85 μmol/L/day, 93.39 μmol/L/day, or 123.93 μmol/L/day.

In specific embodiments, the culture temperature is substantially maintained at 29.5°C or higher, and below or equal to 37°C (i.e., 29.5°C-37°C).

In specific embodiments, the culture temperature is maintained at least 29.5°C, at least 30°C, at least 30.5°C, at least 31°C, at least 31.5°C, at least 32°C, at least 32.5°C, at least 33°C, at least 33.5°C, at least 34°C, at least 34.5°C, at least 35°C, at least 35.5°C, at least 36°C, at least 36.5°C, or at least 37°C.

In specific embodiments, at least 50% to 100% of the time during the protein production phase is maintained at 29.5°C-37°C.

In specific embodiments, the culture temperature is maintained at 29.5°C-37°C for at least 50%, at least 55%, at least 60%, at least 65%, at least 70%, at least 75%, at least 80%, at least 85%, at least 90%, at least 95%, at least 98% or at least 99% of the time during the protein production phase.

In specific embodiments, substantially 95% of the time during the protein production phase is maintained at 29.5°C-37°C.

In specific embodiments, the host cells are mammalian cells.

In specific embodiments, the mammalian cells are derived from a murine or a human.

In specific embodiments, the cells derived from the human include, but are not limited to HEK 293 or PER.C6.

In specific embodiments, the cells derived from the murine include, but are not limited to CHO, Hybridoma, YB2/0, NS0, Sp2/0 or BHK.

In specific embodiments, the host cells are CHO cells.

In a third aspect, the present application provides a pharmaceutical formulation comprising the composition disclosed in the first aspect or the composition prepared according to the method disclosed in the second aspect, and one or more pharmaceutically acceptable excipients.

In a fourth aspect, the present application provides use of the composition disclosed in the first aspect or the composition prepared according to the method disclosed in the second aspect in the preparation of a medicament for the treatment of pulmonary arterial hypertension or skeletal diseases.

In a fifth aspect, the present application provides a method for treating pulmonary arterial hypertension or skeletal diseases, comprising administering to a subject or patient in need thereof a therapeutically effective amount of the composition disclosed in the first aspect, or the composition prepared according to the method disclosed in the second aspect, or the pharmaceutical formulation disclosed in the third aspect.

In a sixth aspect, the present application provides use of iron ions in reducing the content of a sotatercept variant in a sotatercept-containing product, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2, and the sequence of at least one monomeric peptide chain of the variant is as set forth in SEQ ID NO:1.

In a preferred embodiment, the iron ions are derived from ferric citrate.

In a seventh aspect, the present application provides a method for detecting a sotatercept variant in a sotatercept-containing product, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2, and the sequence of at least one monomeric peptide chain of the variant is as set forth in SEQ ID NO:1, and wherein the method comprises:

subjecting the product to lysyl endonuclease (Lys-C) digestion to obtain the digestion product; and
subjecting the digestion product to a peptide mass fingerprinting analysis, for example, by using LC-MS/MS, and determining the presence or absence of the variant based on MS1 and MS2 spectra.

In some embodiments, the method further comprises obtaining XIC chromatograms of the truncated peptide segment and the full-length peptide segment after performing a peptide mass fingerprinting analysis when the variant is determined to be present, and calculating the truncated variant content from the corresponding peak areas.

In an eighth aspect, the present application provides a method for quality inspection or quality control of a sotatercept-containing product, comprising detecting the content of a sotatercept variant in the sotatercept-containing product, wherein the vaiant has a peptide bond cleavage occurred between arginine at position 4 and serine at position 5 (Arg(4)-Ser(5)) in the amino acid sequence (SEQ ID NO:2) of one or two monomeric peptide chains of sotatercept, and the sequence of at least one monomeric peptide chain of the variant is as set forth in SEQ ID NO:1.

In some embodiments, the sotatercept-containing product is the composition disclosed in the first aspect or the pharmaceutical formulation disclosed in the third aspect.

In some embodiments, if the variant content is detected to be less than or equal to about 10%, it indicates that the sotatercept-containing product meets medicinal requirements.

In a ninth aspect, the present application provides use of a sotatercept variant in the quality inspection or quality control of a sotatercept-containing product, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2 , and the sequence of at least one monomeric peptide chain of the variant is as set forth in SEQ ID NO:1.

In some embodiments, the sotatercept-containing product is the composition disclosed in the first aspect or the pharmaceutical formulation disclosed in the third aspect.

## Brief Description of the Drawings

FIG. 1 is a schematic structural representation of sotatercept and the cleavage site of its variant.

FIG. 2 is a deconvolution spectrum of the main components from the total ion chromatogram for analyzing an intact molecular weight of sotatercept.

FIG. 3A, FIG. 3B, and FIG. 3C show the extracted ion chromatogram of MS1, MS1 spectrum, and MS2 spectrum of the sotatercept variant, respectively.

FIG. 4 is a summary graph showing the effects of culture temperature during the protein production phase and average daily addition concentrations of riboflavin, ferric citrate, ammonium metavanadate, and lithium chloride during the culture period on the content of the truncated variant.

FIG. 5 is a trend chart showing the effects of culture temperature during the protein production phase and average daily addition concentrations of riboflavin, ferric citrate, ammonium metavanadate, and lithium chloride during the culture period on the content of the truncated variant.

FIG. 6 is a graph showing the content change of the $R^4/S^5$ truncated variant under different culture temperatures during the protein production phase and different average daily addition concentrations of ferric citrate during the culture period.

FIG. 7 is a graph showing the content change of the $R^4/S^5$ truncated variant under different culture temperatures during the protein production phase.

FIG. 8 is a graph showing the content change of the $R^4/S^5$ truncated variant under different average daily addition concentrations of ferric citrate.

FIGS. 9A, 9B, and 9C show the fitted graphs of binding-dissociation curves for the affinity of sotatercept samples containing different contents of the R4/S5 truncated variant for Activin A, where the ordinate (y axis) is response unit (RU); the abscissa (X axis) is time (seconds), which records the dynamic changes in the process of binding and dissociation.

## Description of the Sequences

SEQ ID NO:1 is a truncated sequence of SEQ ID NO:2, in which the peptide bond between Arg (4)-Ser(5) is cleaved at the N-terminus and four amino acid residues of isoleucine, leucine, glycine and arginine at the N-terminus are deleted, and the specific sequence of SEQ ID NO:1 is as shown below:

SETQECLFFNANWEKDRTNQTGVEPCYGDKDKRRHCFATWKNISGSIEIVKQ
GCWLDDINCYDRTDCVEKKDSPEVYFCCCEGNMCNEKFSYFPEMEVTQPTS
NPVTPKPPTGGGTHTCPPCPAPELLGGPSVFLFPPKPKDTLMISRTPEVTCVVV
DVSHEDPEVKFNWYVDGVEVHNAKTKPREEQYNSTYRVVSVLTVLHQDWL
NGKEYKCKVSNKALPVPIEKTISKAKGQPREPQVYTLPPSREEMTKNQVSLTC
LVKGFYPSDIAVEWESNGQPENNYKTTPPVLDSDGSFFLYSKLTVDKSRWQQ
GNVFSCSVMHEALHNHYTQKSLSLSPGK.

SEQ ID NO:2 is the amino acid sequence of the monomeric peptide chain of sotatercept, and the specific sequence is as shown below:

*ILGR*SETQECLFFNANWEKDRTNQTGVEPCYGDKDKRRHCFATWKNISGSIEI
VKQGCWLDDINCYDRTDCVEKKDSPEVYFCCCEGNMCNEKFSYFPEMEVTQ
PTSNPVTPKPPTGGGTHTCPPCPAPELLGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPVPIEKTISKAKGQPREPQVYTLPPSREEMTKNQVS
LTCLVKGFYPSDIAVEWESNGQPENNYKTTPPVLDSDGSFFLYSKLTVDKSR
WQQGNVFSCSVMHEALHNHYTQKSLSLSPGK.

## DETAILED DESCRIPTION

Sotatercept (CAS No.1001080-50-7) is a homodimer formed from two identical monomeric peptide chains linked by disulfide bonds. The monomeric peptide chain of sotatercept is a fusion peptide chain of an extracellular binding domain of ActRIIA and human Fc, which comprises two parts: one part is an extracellular domain of the human activin receptor type IIA (ActRIIA) to "capture" specific ligands, and another part is the Fc fragment of human immunoglobulin G1 (IgG1) for prolonging the action time of the drug *in vivo*. The extracellular binding domain of ActRIIA and the Fc fragment are fused to form the polypeptide sequence as set forth in SEQ ID NO:2. Specifically, sotatercept is a homodimer formed by two monomeric peptide chains (each is set forth in SEQ ID NO:2) through the disulfide bond between the cysteine residues in the Fc fragment.

The inventors of the present application have unexpectedly found that a sotatercept variant, such as a truncated variant formed by a peptide bond cleavage, has been produced in the preparation of sotatercept, and at least one polypeptide chain of the truncated variant undergoes peptide bond cleavage between the amino acid residues at positions 4 and 5 (Arg(4)-Ser(5)) corresponding to the sequence set forth in SEQ ID NO: 2, which is referred to as the R4/S5 truncated variant. The inventors have unexpectedly found that the peptide bond cleavage between Arg(4) -Ser(5) of less than or equal to about 10% does not affect the binding activity and the biological activity of the sotatercept samples. In other words, as long as the peptide bond cleavage between Arg(4) -Ser(5) does not exceed about 10%, the binding activity and the biological activity of sotatercept will not be reduced. Therefore, in the process of preparing sotatercept, if the cleavage rate of the peptide bond between Arg(4)-Ser(5) is assessed to be no higher than 10%, there is no need to perform operations to remove the variant or impurities. This simplifies the production process to a certain extent and saves production costs.

In specific embodiments, the present application has identified the above sotatercept variant and found that it differs from sotatercept in that the peptide bond of at Arg (4)-Ser(5) at the N-terminus of at least one of its polypeptide chains is cleaved, resulting in the deletion of four N-terminal amino acid residues: isoleucine, leucine, glycine, and arginine. The truncated peptide chain sequence is as set forth in SEQ ID NO: 1. Also referred to herein as $R^4/S^5$ truncated variant.

In some embodiments, the composition described herein consists of sotatercept and the $R^4/S^5$ truncated variant, and the cleavage rate of the peptide bond between Arg(4)-Ser(5) does not exceed 10%.

As used herein, the terms "the cleavage rate of the peptide bond between Arg(4)-Ser(5)", "the cleavage proportion of the peptide bond between Arg(4)-Ser(5)", and "the truncated variant content" can be used interchangeably, and the method for calculating them is the same, and they all refer to the percentage of the monomer peptide chains with peptide bond cleavage between Arg(4)-Ser(5) in the composition relative to the total amount of the monomer peptide chains in the composition, as calculated by a peptide mass fingerprinting analysis.

In some embodiments, liquid chromatography-tandem mass spectrometry (LC-MS/MS) analysis is used to detect the $R^4/S^5$ truncated variant in the sotatercept-containing product. For example, the peptide mass fingerprinting analysis is performed on the test samples using LC-MS/MS.

In specific embodiments, the method for calculating the cleavage rate of the peptide bond between Arg(4)-Ser(5) based on the peptide mass fingerprinting analysis comprises the following steps: centrifuging after enzymatic digestion of the sample, collecting the supernatant, subjecting the test sample to the peptide mass fingerprint analysis by using the liquid chromatography-tandem mass spectrometry (LC-MS/MS) to obtain mass spectrum data, extracting the extracted ion chromatograms (XIC) of two charge forms which have the strongest responses of the truncated peptide segment and its corresponding full-length peptide segment, integrating to obtain respective peak areas, and calculating the content (i.e., the cleavage rate of the peptide bond between Arg(4)-Ser(5)) of the $R^4/S^5$ truncated variant according to the following formula:

The content of $R^4/S^5$ truncated variant=(XIC area of truncated peptide segment)/(XIC area of truncated peptide segment+XIC area of full-length peptide segment) $\times$ 100%.

In a more specific embodiment, the method for calculating the cleavage rate of the peptide bond between Arg(4)-Ser(5) based on the peptide mass fingerprinting analysis comprises the following: taking a sample, denaturing the sample with urea and reducing it with TCEP, then alkylating it with iodoacetamide(IAM), adding ammonium bicarbonate buffer and then adding lysine protease at a ratio of protein: enzyme = 20: 1 (mass ratio) for digestion at 37°C for 15h, and adding formic acid at a final concentration of 1% to terminate the digestion after the enzymatic digestion is completed, and centrifuging at 12,000 rpm for 3 min, and transferring the supernatant into a sample vial; subjecting the test sample to the peptide mass fingerprint analysis by using the liquid chromatography-tandem mass spectrometry (LC-MS/MS) to obtain mass spectrum data, extracting the extracted ion chromatograms (XIC) of two charge forms which have the strongest responses of the truncated peptide segment and its corresponding full-length peptide segment, integrating to obtain respective peak areas, and calculating the content (i.e., the cleavage rate of the peptide bond between Arg(4)-Ser(5)) of the $R^4/S^5$ truncated variant according to the following formula:

The content of $R^4/S^5$ truncated variant=(XIC area of truncated peptide segment)/(XIC area of truncated peptide segment+XIC area of full-length peptide segment) $\times$ 100%.

In specific embodiments, sotatercept is expressed in a suitable host cell. Nonlimiting examples of such host cell include, but are not limited to, CHO, DUK-B11, NS0, Sp2/0, BHK, HEK 293, BHK, and the like.

In specific embodiments, the CHO cells used are selected from CHO-S, CHOZN, CHO K1, CHO DG44, and the like.

In some embodiments, cells capable of expressing sotatercept are cultured in a bioreactor, and the culturing includes a cell expansion phase and a protein production phase. Preferably, the culture temperature during the protein production phase is substantially maintained at about 29.5°C-37°C.

In specific embodiments, the protein production phase refers to a process from the time of the first feeding in fed-batch culture±0 day, ±0.5 day, ±1 day, ±1.5 day, ±2 day, 2.5 day, ±3 day, ±3.5 day, ±4 day, ±4.5 day, ±5 day, ±5.5 day, ±6 day, ±6.5 day, ±7 day, ±7.5 day, ±8 day, ±8.5 day, ±9 day, ±9.5 day, ±10 day, ±10.5 day, ±11 day, ±11.5 day, ±12 day, ±12.5 day, ±13 day, ±13.5 day, ±14 day, ±14.5 day, ±15 day, ±15.5 day, ±16 day, ±16.5 day, ±17 day, ±17.5 day, ±18 day, ±18.5 day, ±19 day, ±19.5 day, or ±20 day to the end of the culture, or a process from the time of initiating medium perfusion in the perfusion culture ±0 day, ±0.5 day, ±1 day, ±1.5 day, ±2 day, 2.5 day, ±3 day, ±3.5 day, ±4 day, ±4.5 day, ±5 day, ±5.5 day, ±6 day, ±6.5 day, ±7 day, ±7.5 day, ±8 day, ±8.5 day, ±9 day, ±9.5 day, ±10 day, ±10.5 day, ±11 day, ±11.5 day, ±12 day, ±12.5 day, ±13 day, ±13.5 day, ±14 day, ±14.5 day, ±15 day, ±15.5 day, ±16 day, ±16.5 day, ±17 day, ±17.5 day, ±18 day, ±18.5 day, ±19 day, ±19.5 day, or ±20 day to the end of the culture.

The inventors of the present application have found that the content of the variant in the sotatercept sample is affected by the culture temperature during the protein production phase.

In specific embodiments, the culture temperature is maintained at 29.5°C or higher and below or equal to 37°C (i.e., 29.5°C-37°C) during the protein production phase.

In specific embodiments, the culture temperature is substantially maintained at, for example, about 30°C ± 0.5°C, about 32°C ± 0.5°C, about 34°C ± 0.5°C, or about 36.5°C ± 0.5°C, or any value between 29.5°C-37°C.

In specific embodiments, at least 50% to 100% of the time during the protein production phase is maintained at 29.5°C-37°C.

In specific embodiments, the culture temperature is maintained at 29.5°C-37°C for at least 50%, at least 55%, at least 60%, at least 65%, at least 70%, at least 75%, at least 80%, at least 85%, at least 90%, at least 95%, at least 98% or at least 99% of the time during the protein production phase.

In specific embodiments, the 95% time of the protein production phase is maintained at 29.5°C-37°C.

In specific embodiments, the culture temperature during the protein production phase is selected from 30°C, 32°C, 34°C or 36.5°C or the like.

In specific embodiments, as the culture temperature during the protein production phase gradually decreases, the truncated variant content gradually decreases.

In some embodiments, the inventors of the present application have found that the content of the variant in the sotatercept sample is affected by iron ions such as ferric citrate in the culture medium.

In specific embodiments, the average daily addition concentration of iron ions in the culture medium during the culture period is from 1.5 μmol/L/day to 125 μmol/L/day.

In specific embodiments, the above culture period refers to the entire culture period including the cell expansion phase and the protein production phase.

In specific embodiments, using ferric citrate as a source of iron ions, an exemplary formula for calculating average daily addition concentration is:

Average daily addition concentration of ferric citrate (μmol/L/day) = (total addition amount of ferric citrate in culture medium(μmol))/(harvested culture volume (L) × culture days(day)).

The "total addition amount" in the above formula, in simple terms, refers to the cumulative amount of ferric citrate added to the bioreactor in any form throughout the entire bioreactor culture process. Specifically, the total amount includes the amount of ferric citrate already contained in the cell culture when it is inoculated into the bioreactor (which may also be zero), the amount of ferric citrate added to the bioreactor in advance (which may also be zero), the amount of ferric citrate in the feed medium throughout the entire culture process, and the amount of ferric citrate added in other forms.

In specific embodiments, the average daily addition concentration of the iron ions is 1.50 μmol/L/day to 5.00 μmol/L/day, 5.01 μmol/L/day to 15.00 μmol/L/day, 15.01 μmol/L/day to 30.00 μmol/L/day, 30.01 μmol/L/day to 45.00 μmol/L/day, 45.01 μmol/L/day to 60.00μmol/L/day, 60.01 μmol/L/day to 75.00 μmol/L/day, 75.01 μmol/L/day to 90.00 μmol/L/day, 90.01 μmol/L/day to 115.00 μmol/L/day, or 115.01 μmol/L/day to 125.00 μmol/L/day, or any interval within any of the above ranges or any value within the interval.

In specific embodiments, the average daily addition concentration of iron ions is selected from 1.77 μmol/L/day, 7.88 μmol/L/day, 62.85 μmol/L/day, 93.39 μmol/L/day, or 123.93 μmol/L/day.

In specific embodiments, the sample loading phase is initiated after equilibration of the affinity chromatography column with a salt-containing buffer. After the end of the sample loading, the salt-containing buffer is further used for rinsing, followed by rinsing with a salt-free buffer, and eluting with a buffer having a pH of 3.0~4.0, and an eluted protein is collected.

In specific embodiments, the content of the variant in the composition comprising sotatercept and its variant disclosed herein is about 0.01% to about 10%, about 0.05% to about 10%, about 0.1% to about 10%, about 0.2% to about 10%, about 0.3% to about 10%, about 0.4% to about 10%, about 0.5% to about 10%, about 0.6% to about 10%, about 0.7% to about 10%, about 0.8% to about 10%, about 0.9% to about 10%, about 1% to about 10%, about 1.5% to about 10%, about 2% to about 10%, about 2.5% to about 10%, about 3% to about 10%, about 3.5% to about 10%, about 4% to about 10%, about 4.5% to about 10%, about 5% to about 10%, about 5.5% to about 10%, about 6% to about 10%, about 6.5% to about 10%, about 7% to about 10%, about 7.5% to about 10%, about 8% to about 10%, about 8.5% to about 10%, about 9% to about 10%, about 9.5 to about 10%, or any interval within any of the above ranges or any value within the interval.

In a more specific embodiment, the content of the variant is 0.3%-9%, for example 0.33%-8.58%, or any interval within any of the above ranges or any value within the interval.

Also disclosed herein is the preparation of a pharmaceutical formulation of the composition disclosed herein, i.e., the composition is mixed with an optional pharmaceutically acceptable excipient and stored in the form of a lyophilized formulation or an aqueous solution. Pharmaceutically acceptable excipients are nontoxic to recipients at the dosages and concentrations employed. Pharmaceutically acceptable excipients include pharmaceutically acceptable carriers and/or adjuvants.

In some embodiments, pharmaceutically acceptable carriers and/or adjuvants include, but are not limited to, diluents, binders, surfactants, humectants, adsorbent carriers, lubricants, and/or disintegrants. Among them, the diluents include, but are not limited to, lactose, sodium chloride, glucose, urea, starch and water; the binders include, but are not limited to, starch, pregelatinized starch, dextrin, maltodextrin, sucrose, acacia, gelatin, methylcellulose, carboxymethylcellulose, ethylcellulose, polyvinyl alcohol, polyethylene glycol, polyvinylpyrrolidone, alginic acid, and alginates, xanthan gum, hydroxypropylcellulose, and hydroxypropylmethylcellulose; surfactants include, but are not limited to, polyoxyethylene sorbitan fatty acid esters, sodium dodecyl sulfate, monoglyceride stearate, cetyl alcohol; the humectants include, but are not limited to, glycerol and starch; the adsorbent carriers include, but are not limited to, starch, lactose, bentonite, silica gel, kaolin, and soap clay; the lubricants include, but are not limited to, zinc stearate, glycerol monostearate, polyethylene glycol, talc, calcium and magnesium stearate, polyethylene glycol, boric acid powder, hydrogenated vegetable oil, sodium stearyl fumarate, polyoxyethylene monostearate, sucrose monolaurate, sodium lauryl sulfate, magnesium lauryl sulfate, and magnesium dodecyl sulfate. In some embodiments, the pharmaceutical formulation is pyrogen-free.

In some embodiments, the pharmaceutical formulation disclosed herein includes dosage forms for parenteral and non-parenteral administration.

In specific embodiments, the dosage forms for parenteral administration include solutions, drops, tablets, capsules, granules, films, gels, powders, emulsions, suspensions, dropping pills, suppositories, aerosols, sprays, powder inhalation, patches, ointments or creams.

In specific embodiments, the dosage forms for non-parenteral administration include dosage forms for injection administration, dosage forms for respiratory administration, dosage forms for cavity administration, dosage forms for mucosal administration, or dosage forms for dermal administration.

In specific embodiments, the dosage forms for injection administration include, but are not limited to, intravenous, intramuscular, subcutaneous, intradermal, and intracavitary injections, and the like; the dosage forms for respiratory administration include, but are not limited to, sprays, aerosols, powder inhalation, etc.; the dosage forms for cavity administration include, but are not limited to, suppositories, aerosols, effervescent tablets, drops, dropping pills, etc. for rectum, vagina, urethra, nasal cavity, ear canal, etc.; the dosage forms for mucosal administration include, but are not limited to, eye drops, nose drops, ophthalmic ointments, gargles, sublingual tablets, adhesive tablets, film patches, etc.; and the dosage forms for dermal administration include, but are not limited to, topical solutions, lotions, liniments, ointments, plasters, pastes, patches, etc.

It is described herein that cell culture media are used to prepare sotatercept. In some embodiments, the cell culture medium is a chemically defined medium ("CDM").

In the present specification and claims, the terms "culture medium" and "cell culture medium" refer to a nutrient source used to grow or maintain cells. It will be appreciated by those skilled in the art that the nutrient source may contain ingredients necessary for cell growth and/or survival, or may contain ingredients that facilitate cell growth and/or survival. Vitamins, essential or non-essential amino acids, and trace elements are ingredients of the culture medium.

The term "chemically defined medium" refers to a medium that does not contain products of animal origin, such as animal serum and peptone. The term also encompasses a culture medium with a specified composition, free of undefined or partially defined components (e.g., ingredients such as animal serum, animal peptone, and plant peptone).

In this specification and in the claims, the term "about" or "substantially" refers to a numerical range considered by one of ordinary skill in the art to be equivalent to the recited numerical value (e.g., having the same function or result), e.g., +/-10% of the recited numerical value.

In the present specification and claims, the term "impurity" refers to a substance different from the polypeptide product of interest. Impurities include, but are not limited to, host cell materials, such as host protein; nucleic acids; desired variants, fragments, aggregates or derivatives of proteins and polypeptides; other polypeptides; endotoxins; viral contaminants; cell culture medium components, etc.

In this specification and in the claims, the phrases "include", "comprise" and "contain" mean "include but are not limited to", and does not intended to exclude other parts, additives, components or steps.

It should be understood that features, characteristics, components, or steps described in a particular aspect, embodiment, or example of the present application can be applied to any other aspect, embodiment, or example described herein unless contradicted thereby.

The above disclosure generally describes the present application, which will be further exemplified by the following Examples. These Examples are described merely to illustrate the application and are not intended to limit the scope of the application. Although specific terms and values are used herein, such terms and values are also understood to be exemplary and do not limit the scope of the application. Unless otherwise specified, the experimental methods and techniques in this specification are those well known to a person skilled in the art.

## EXAMPLES

### Example 1. Preparation of Sotatercept

(1) Construction of recombinant plasmid of monoclonal antibody

The amino acid sequence of sotatercept was as set forth in SEQ ID NO:2, and the gene synthesis company was entrusted to synthesize the complete sequence and the gene sequence was linked to the multiple cloning site (MCS) of the corresponding vector. After the sequence was verified to be correct by sequencing, the plasmid was extracted using the Midiprep plasmid kit (purchased from MN, Germany, Item Number 740422.50) and stored at -80°C for later use.

(2) Resuscitation and shake flask culture of host cells

The host cells used in this study were CHOZN ZFN-Modified $GS^{-/-}$ CHO cell lines (hereinafter referred to as "CHOZN cells") purchased from Merck. The CHOZN cells used in the present application were taken from liquid nitrogen, rapidly thawed in a water bath at 37°C, added to the preheated EX-CELL CD CHO Fusion medium on an ultraclean workbench, and cultured in an infors shaker.

(3) Cell transfection

After counting on the day of transfection, the cells to be transfected were mixed with plasmids and transfected by electroporation of exponential waves using a BioRad electroporator. After the electric shock, the cells were immediately mixed with preheated culture medium and transferred to a carbon dioxide incubator for static culture.

(4) Cell pool screening

After standing culture of 24 hours following electroporation, cell pools were screened using a mixed culture of 80% EX-CELL CHO Cloning medium and 20%EX-CELL CD CHO Fusion medium with an initial seeding density of 3,000-10,000 cells/well. After inoculation, 96-well plates were placed in a carbon dioxide incubator for standing incubation. After the cells were 100% confluency in the wells, the expression level was evaluated by Octet, and the cell pools with high expression levels were selected and expanded to 24-well plates. The cell growth was observed every 2-5 days, and after the cell density was high and the state was good, the cell count and yield were evaluated, and the cell pools with high yield were screened for inoculation of monoclonal cell strains based on the results of cell number and yield.

(5) Screening of monoclonal cell strains

After monoclonal sorting, the cell pools with high expression amounts were placed in 96-well plates for culture, and then the monoclonal cell imaging was confirmed every day, the expression level was evaluated by Octet after the cells were 100% confluency in the wells, and the cell pools with high expression levels were selected and expanded to 24-well plates. The cell growth was observed every 2-5 days, and after the cell density was high and the state was good, the cell count and yield were evaluated. The monoclonal cell strains with high expression levels were screened for evaluation of shake flask Fed-Batch culture based on the results of cell number and yield, with indicators such as expression level and product quality assessed.

(6) Fed batch culture experiment

With reference to the typical cell culture process of antibody-based drugs, the production of sotatercept was carried out according to the process of cell resuscitation, step-by-step expansion culture of shake flasks and bioreactors, and production by production tanks, wherein a fed-batch culture process was employed in the production phase. A cell cryopreservation tube was taken and thawed in a water bath at 37°C, the cells were cultured in shake flask expansion medium. After successive expansion culture in a shake flask and a bioreactor, the cells are inoculated into a production tank for production. With reference to the typical cell culture process of antibody-based drugs, process parameters such as suitable culture temperature, pH, dissolved oxygen, stirring speed, and aeration were set in the production phase, an appropriate feeding medium and glucose solution were added according to cell growth requirements, and an antifoaming agent solution was added according to culture requirements. After completion of the culture, the cell culture solution was subjected to deep filtration and sterilization filtration, and the supernatant was collected and subjected to one-step ProA affinity chromatography purification.

**Example 2. Discovery of $R^4/S^5$ Truncated Variant in Sotatercept Sample**

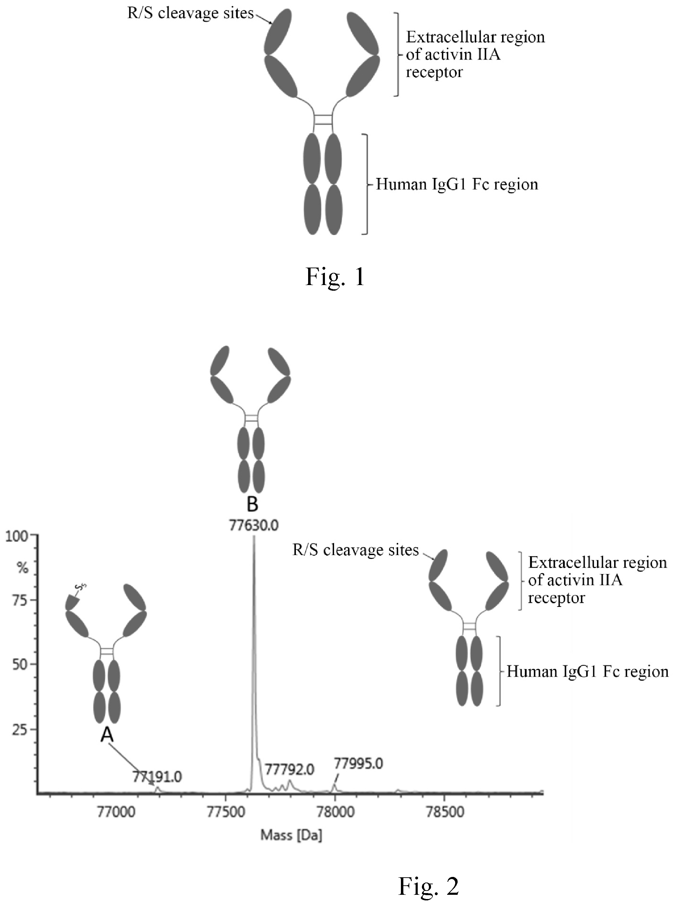

The structure of sotatercept prepared in Example 1 was shown in Figure 1.
The sample prepared in Example 1 was subjected to complete molecular weight analysis. As sotatercept contained 6 N-glycosylation modification sites and 8 O-glycosylation modification sites, the heterogeneity caused by N-glycosylation and O-glycosylation was very serious, and the complete molecular weight cannot be determined without cleaving N-sugar chain and O-sugar chain. Therefore, the sample was treated with OmniGLYZOR immobilized enzymes of GENOVIS for N-sugar and O-sugar cleavage prior to the analysis of complete molecular weight. The method for analyzing complete molecular weight by cleavage of N-sugar and O-sugar comprises the following steps.

100 μg protein was added to the column and the column was sealed with a top cap. The column was placed upright (to prevent resin contact with the top cap) in a shaker at 37°C and incubated at 300 rpm for 4 h; after the incubation, the bottom cap was removed, the column was placed in a collection tube, the top cap was released, the collection tube was centrifuged at 1000×g for 1 min, and the deglycosylated protein was recovered. To maximize sample collection, the column was sealed with a bottom cap, 100 μl reaction buffer was added, and the column was sealed to allow complete suspension of the medium. The bottom cap was removed, the column was placed in a collection tube, the top cover was released, the collection tube was centrifuged at 1000×g for 1 min, and the deglycosylated protein was recovered. The recovered deglycosylated protein was concentrated to a volume of about 50 μl using a centrifugal concentrator, and the protein content of the sample was determined using Lunatic (extinction coefficient: 1.51).

Complete molecular weight analysis of the test sample was carried out using liquid chromatography-mass spectrometry (LC-MS), and the conditions used for chromatography were shown in Table 1 and the conditions used for mass spectrometry were shown in Table 2.

Table 1: Conditions for chromatography

| Name | Conditions |
|---|---|
| LC system | ACQUITY I-Class, Waters |
| Chromatography column | ACQUITY UPLC Protein BEH SEC, 1.7 μm, 4.6 mm×150 mm |
| Column temperature | Room temperature |
| Sample chamber temperature | 8°C |
| Mobile phase | phase A: a solution of 0.1% formic acid /water; phase B: a solution of 0.1% formic acid /acetonitrile; |
| Elution gradient | phase A: 80%, phase B:20% |

Table 2: Conditions for mass spectrometry

| Name | Conditions |
|---|---|
| MS system | Xevo G2-XS QTof, Waters |
| Ionization mode | ESI positive |
| MS1 scan range | 500-4000 m/z |
| Capillary voltage | 2.7 kV |
| Cone voltage | 150 V |
| Ion source temperature | 100°C |
| Desolvation gas temperature | 400°C |
| Cone gas velocity | 50 L/h |
| Desolvation gas flow rate | 800 L/h |

Data processing was performed using UNIFI 1.8 software of Waters company. The deconvolution spectrum of the main components from the total ion chromatogram (TIC) for analyzing intact molecular weight of sotatercept was shown in FIG. 2.

The N-sugar chain and the O-sugar chain on the protein were both released by digestion after the sample was digested with the OmniGLYZOR immobilized enzyme of GENOVIS, and after deconvolution of the TIC, it was found that the molecular weight of the main peak (peak B) was consistent with the molecular weight of the protein backbone (without N-sugar and O-sugar modification), and the error between the observed molecular weight and theoretical molecular weight was not higher than 5.0 Da. The molecular weight of the peak (peak A) in front of the main peak was about 439 Da less than that of the main peak, which is presumed to be related to protein truncation. Combined with the sequence analysis of sotatercept, the theoretical molecular weight of the remaining component after truncating I1-R4 in one chain of the ActRIIA region was 77190.2 Da, which was similar to the observed molecular weight of peak A.

**Example 3. Identification of $R^4/S^5$ Truncated Variant in Sotatercept Sample**

To confirm the cleavage site, the sample prepared in Example 1 was subjected to the peptide mass fingerprinting analysis. The analytical procedure was as follows.

About 100 μg of the sample was added with a final concentration of 6 M of urea and 10 mM of TCEP, and incubated at 37°C for 60 min for denaturation and reduction; and IAM at a final concentration of 20 mM was added and incubated at 37°C for 15 min for alkylation to block free sulfhydryl groups.

The urea concentration was diluted to 1 M by the addition of 50 mM ammonium bicarbonate buffer, and lysine endopeptidase (Lys-C) was added at a ratio of protein: enzyme = 20: 1 (mass ratio) for digestion at 37°C for 15 h; and formic acid at a final concentration of 1% was added to terminate the digestion after the enzymatic digestion is completed, and the sample was centrifuged at 12,000 rpm for 3 min, and the supernatant was transferred into a sample vial; and the test sample was subjected to the peptide mass fingerprint analysis by using the liquid chromatography-tandem mass spectrometry (LC-MS/MS), the conditions used for chromatography was as shown in Table 3 and the conditions used for mass spectrometry was as shown in Table 4.

Table 3: Conditions for chromatography used to identify $R^4/S^5$ truncated variant in sotatercept

| Name | Conditions |
|---|---|
| LC system | Vanquish Flex UHPLC |
| Chromatography column | Acquity UPLC Peptide BEH $C_{18}$ Column, 130Å, 1.7 μm, 2.1 mm×100 mm |
| Column temperature | 60°C |
| Sample chamber temperature | 8°C |
| Mobile phase | phase A: a solution of 0.1% formic acid /water; phase B: a solution of 0.08% formic acid/80% acetonitrile/20% aqueous solution; |
| Elution gradient | After equilibrating for 3 minutes with 1% phase B, phase B linearly rises to 45% within 57 minutes |

Table 4: Conditions for mass spectrometry used to identify $R^4/S^5$ truncated variant in Sotatercept

| Name | Conditions |
|---|---|
| Mass spectrometry system | Exploris 480, Thermo |
| Acquisition mode | Positive ion mode |
| Capillary Temperature for ion transmission | 320°C |
| Spray voltage | 3.2 kV |
| MS1 resolution | 90000 |
| MS1 AGC Target | 1e6 |
| MS1 scan range | 200-2000 m/z |
| MS2 resolution | 15000 |
| MS2 AGC Target | 1e5 |
| Selection of ion width | 1.6 m/z |

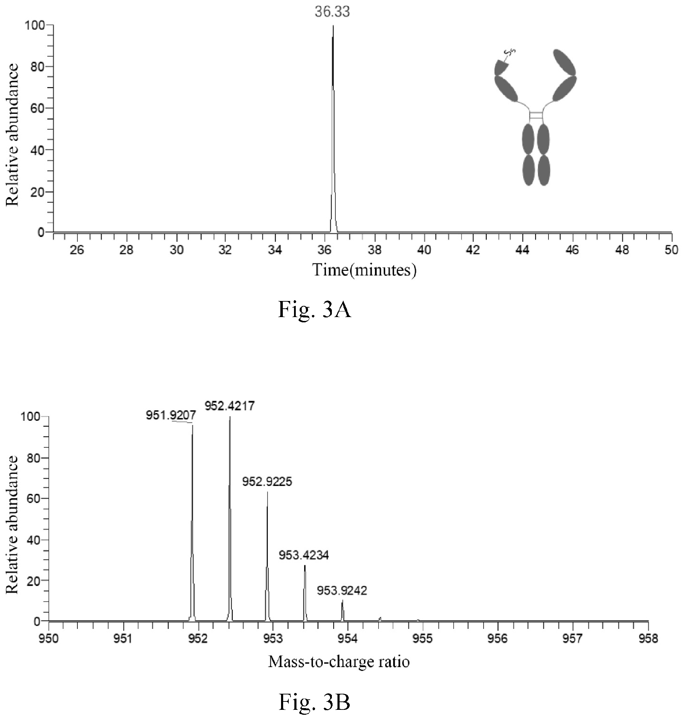

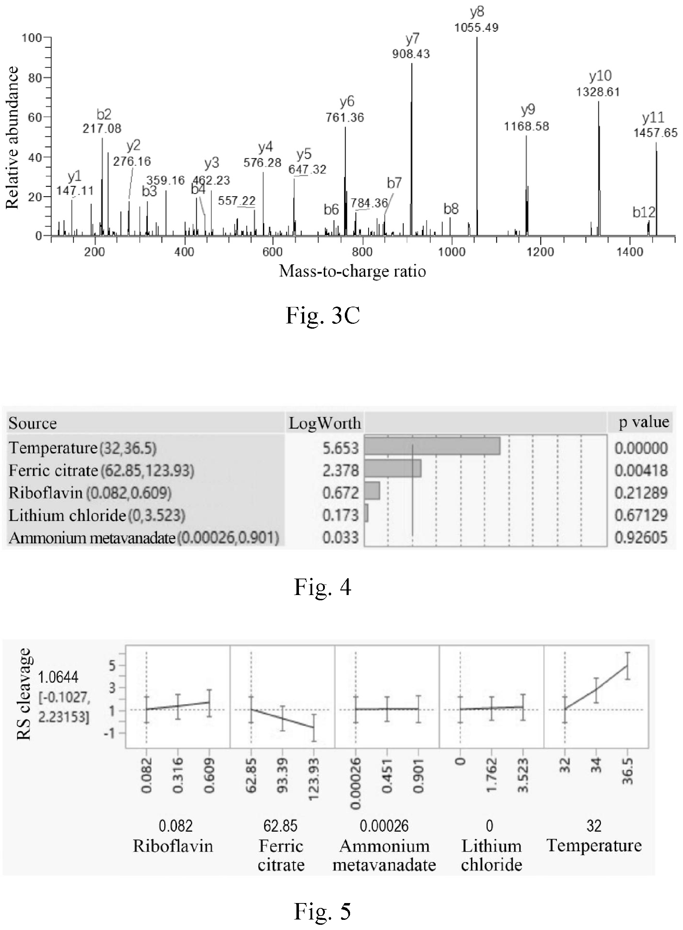

Data processing was performed using pFind software. The extracted ion chromatogram (XIC) of MS1, MS1 spectrum, and MS2 spectrum of the truncated peptide segment, were shown in FIGS. 3A, 3B and 3C, respectively. The results showed that a truncated peptide segment $^{4}S^{5}$ETQECLFFNANWEK (in which the deleted amino acid residues were marked by strikethrough) was found at the retention time of 44.3 min in the sample. The observed monoisotopic peak m/z of the peptide fragment was 951.9207, the theoretical m/z was 951.9193, with an error of only 1.47 ppm. Meanwhile in the MS2 spectrum, the fragment ions can be well matched with the theoretical b and y ions of the peptide fragment, thereby confirming the amino acid sequence of the peptide fragment. Sotatercept was a homodimer formed from two identical peptide chains linked by disulfide bonds, and in combination with the results of complete molecular weight analysis, peak A was formed by the cleavage of the C-terminal peptide chain at the $R^4$ site of one peptide chain, resulting in the deletion of I1-R4 in the ActRIIA region. Peak A had an observed molecular weight of 77191.0 Da and the theoretical molecular weight of the remaining component after truncating I1-R4 at the N-terminus of one chain in the ActRIIA region was 77190.2 Da, further demonstrating that the cleavage site was the $R^4$ site of one peptide chain.

**Example 4. Content of Truncated Variant in Sotatercept Sample**

Sotatercept was prepared according to the preparation method in Example 1, and the obtained sample was identified according to the identification method in Example 3, and the content of the $R^4/S^5$ truncated variant was calculated according to the following formula:

The content of $R^4/S^5$ truncated variant=(XIC area of truncated peptide segment)/(XIC area of truncated peptide segment+XIC area of full-length peptide segment)×100%.

The sample pre-processing method and the LC-MS/MS detection method were as shown in Example 2, and data processing was performed using FreeStyle software of Thermo. The content of the $R^4/S^5$ truncated variant in the batch 1 sample was calculated according to the formula and the content of 3.45% was obtained by the following calculation:

The content of $R^4/S^5$ truncated variant=(524498755+4110334)/ (524498755+4110334+3966356900 +10827935806)×100%.

The contents of the $R^4/S^5$ truncated variants in multiple batches of samples were calculated in the same manner, and the results were shown in Table 5.

Table 5. Contents of truncated variants in multiple batches of sotatercept samples

| Batch information | Content of $R^4/S^5$ truncated variant (%) |
|---|---|
| Batch 1 | 3.45 |
| Batch 2 | 2.17 |
| Batch 3 | 3.13 |

**Example 5. Effects of Average Daily Addition Concentrations of Riboflavin, Ferric Citrate, Ammonium Metavanadate and Lithium Chloride during the Culture and Culture Temperature in the Protein Production phase on the Contents of Truncated Variant**

The cell culture process of host cells containing a nucleic acid molecule encoding an amino acid sequence as set forth in SEQ ID NO:2 or a vector comprising the nucleic acid molecule referred to the method described in Example 1, and the effects of average daily addition concentrations of riboflavin, ferric citrate, ammonium metavanadate and lithium chloride during the culture and culture temperature in the protein production phase on the content of the truncated variant were tested. After completion of the culture, the culture solution was collected at one time and subjected to one-step Protein A affinity chromatography, and the obtained samples were identified according to the identification method in Example 3. The truncated variant contents obtained under different average daily addition concentrations of riboflavin, ferric citrate, ammonium metavanadate and lithium chloride during the culture and different culture temperature in the protein production phase were shown in Table 6.

Table 6. Contents of the truncated variant obtained under different average daily addition concentrations of riboflavin, ferric citrate, ammonium metavanadate and lithium chloride during the culture and different culture temperature in the protein production phase

| No. | Riboflavin (μmol/L/day) | Ferric citrate (μmol/L/day) | Ammonium metavanadate (μmol/L/day) | Lithium Chloride (μmol/L/day) | Culture temperature( °C) | Content of $R^4/S^5$ truncated variant(%) |
|---|---|---|---|---|---|---|
| 1 | 0.082 | 62.85 | 0.00026 | 3.523 | 36.5 | 5.17 |
| 2 | 0.082 | 62.85 | 0.451 | 0 | 34 | 2.25 |
| 3 | 0.082 | 93.39 | 0.00026 | 1.762 | 32 | 0.33 |
| 4 | 0.082 | 93.39 | 0.901 | 3.523 | 36.5 | 3.2 |

(continued)

| No. | Riboflavin (μmol/L/day) | Ferric citrate (μmol/L/day) | Ammonium metavanadate (μmol/L/day) | Lithium Chloride (μmol/L/day) | Culture temperature( °C) | Content of $R^4/S^5$ truncated variant(%) |
|---|---|---|---|---|---|---|
| 5 | 0.082 | 123.93 | 0.451 | 1.762 | 32 | 0.34 |
| 6 | 0.082 | 123.93 | 0.901 | 0 | 34 | 0.6 |
| 7 | 0.316 | 62.85 | 0.901 | 0 | 32 | 1.49 |
| 8 | 0.316 | 62.85 | 0.901 | 1.762 | 36.5 | 7.23 |
| 9 | 0.316 | 93.39 | 0.00026 | 0 | 32 | 0.78 |
| 10 | 0.316 | 93.39 | 0.451 | 3.523 | 34 | 2.26 |
| 11 | 0.316 | 123.93 | 0.00026 | 3.523 | 34 | 1.68 |
| 12 | 0.316 | 123.93 | 0.451 | 1.762 | 36.5 | 4.16 |
| 13 | 0.609 | 62.85 | 0.00026 | 1.762 | 34 | 3.47 |
| 14 | 0.609 | 62.85 | 0.451 | 3.523 | 32 | 1.32 |
| 15 | 0.609 | 93.39 | 0.451 | 0 | 36.5 | 4.12 |
| 16 | 0.609 | 93.39 | 0.901 | 1.762 | 34 | 2.37 |
| 17 | 0.609 | 123.93 | 0.00026 | 0 | 36.5 | 3.85 |
| 18 | 0.609 | 123.93 | 0.901 | 3.523 | 32 | 0.65 |
| 19 | 0.082 | 62.85 | 0.00026 | 0 | 36.5 | 4.91 |
| 20 | 0.082 | 7.88 | 0.00026 | 0 | 36.5 | 5.76 |
| 21 | 0.082 | 1.77 | 0.00026 | 0 | 36.5 | 8.58 |
| 22 | 0.316 | 62.85 | 0.451 | 1.762 | 36.5 | 6.37 |
| 23 | 0.316 | 62.85 | 0.451 | 1.762 | 32 | 1.29 |
| 24 | 0.316 | 62.85 | 0.00026 | 0 | 36.5 | 5.90 |
| 25 | 0.316 | 62.85 | 0.00026 | 0 | 34 | 3.74 |
| 26 | 0.316 | 62.85 | 0.00026 | 0 | 32 | 1.76 |
| 27 | 0.316 | 62.85 | 0.00026 | 0 | 30 | 0.38 |
| Note: the control accuracy of culture temperature was within the range of ±0.5°C. | | | | | | |

Data processing was performed using the DOE experimental design software, and the summary result of the effects was shown in FIG. 4, and the influence trend was shown in FIG. 5, where the average daily addition concentration of ferric citrate during the culture and the culture temperature in the protein production phase had a significant effect on the truncated variant content, whereas the average daily addition concentration of riboflavin, the average daily addition concentration of ammonium metavanadate, and the average daily addition concentration of lithium chloride had no significant effect on the truncated variant content.

Among them, as the culture temperature in the protein production phase gradually decreased, the content of the sotatercept variant in the sotatercept sample gradually decreased. Moreover, as the average daily addition concentration of ferric citrate gradually increased during the culture, the content of the sotatercept variant in the sotatercept sample also gradually decreased.

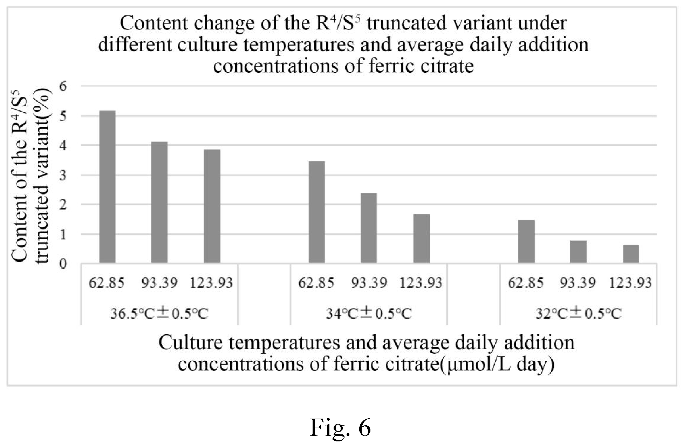

In a further analysis of Example 5, the applicant found that the change in the contents of the $R^4/S^5$ truncated variants under different culture temperatures in the protein production phase and the average daily addition concentrations of ferric citrate during the culture was shown in FIG. 6, irrespective of the average daily addition concentration of riboflavin, the average daily addition concentration of ammonium metavanadate, and the average daily addition concentration of lithium chloride, which had no effect on the truncated variant content.

The results showed that the content of the sotatercept variant in the sotatercept sample was affected by the culture temperature in the protein production phase and the average daily addition concentration of ferric citrate during the culture. The truncated variant content gradually decreased with the gradual decrease of the culture temperature in the protein production phase and the gradual increase of the average daily addition concentration of ferric citrate during the culture. It was expected that the truncated variant content can be further reduced after the downstream purification process.

**Example 6. Effect of Culture Temperature during Protein Production phase on the Content of $R^4/S^5$ Truncated Variants**

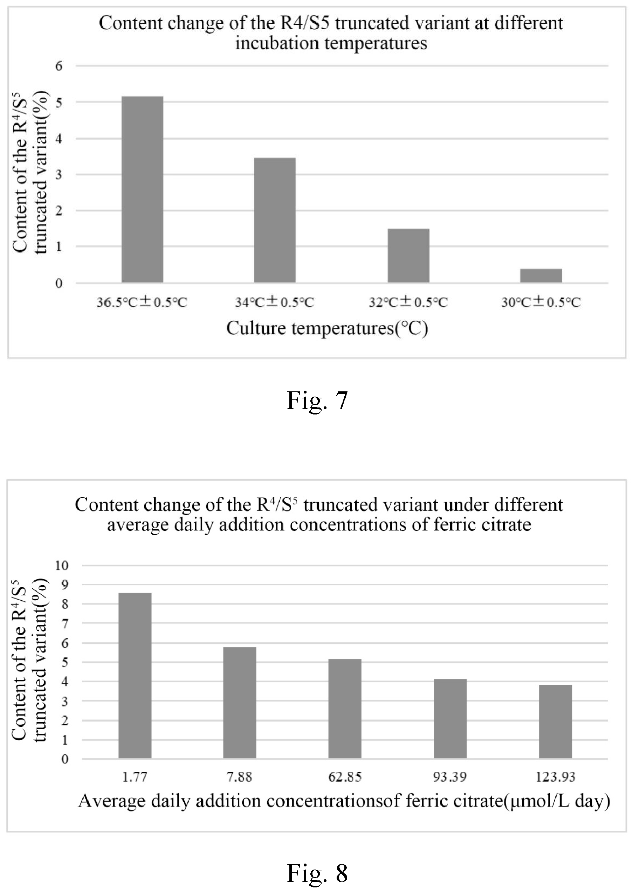

The cell culture process of host cells comprising a nucleic acid molecule encoding an amino acid sequence as set forth in SEQ ID NO:2 or a vector comprising the nucleic acid molecule referred to the method in Example 1, and the effect of culture temperature on the truncated variant content in the protein production phase was further tested. After completion of the culture, the culture solution was collected at one time and subjected to one-step Protein A affinity chromatography, and the obtained samples were identified according to the identification method in Example 3. The content change of the $R^4/S^5$ truncated variant under different culture temperatures during the protein production phase was shown in FIG. 7. The results showed that the content of the sotatercept variant in sotatercept sample was affected by the culture temperature during the protein production phase. As the culture temperature during the protein production phase gradually decreased, the truncated variant content gradually decreased. Moreover, the use of culture temperature of 29.5°C-37°C during the protein production phase can control the content of the sotatercept variant below 10%, and it was expected that the sotatercept variant content can be further greatly reduced after the downstream purification process.

**Example 7. Effect of Average Daily Addition Concentration of Iron Ions on the Content of $R^4/S^5$ Truncated Variants during the Culture**

The cell culture process of host cells comprising a nucleic acid molecule encoding an amino acid sequence as set forth in SEQ ID NO:2 or a vector comprising the nucleic acid molecule referred to the method in Example 1, and the effect of the average daily addition concentration of iron ions on the truncated variant content during the culture was further tested.
In this example and in Example 5, ferric citrate was used as a source of iron ions, and the average daily addition concentration of ferric citrate was calculated as follows:

Average daily addition concentration of ferric citrate (μmol/L/day) = (total addition amount of ferric citrate in culture medium(μmol))/(harvested culture volume (L) × culture days(day)).

After completion of the culture, the culture solution was collected at one time and subjected to one-step Protein A affinity chromatography, and the obtained samples were identified according to the identification method in Example 3. The content change of the $R^4/S^5$ truncated variant at different average daily addition concentrations of ferric citrate was shown in FIG. 8.
The results showed that the content of the sotatercept variant in sotatercept sample was affected by the average daily addition concentration of iron ions during the culture, and as the average daily addition concentration of iron ions gradually increased, the truncated variant content gradually decreased. Moreover, maintaining the average daily addition concentration of iron ions within the range of 1.77-123.93 μmol/L/day can control the content of the sotatercept variant below 10%, and it was expected that the sotatercept variant content can be further significantly reduced after the downstream purification process.

**Example 8. Effect of Different Contents of $R^4/S^5$ Truncated Variants on Activin A Affinity**

The affinity of sotatercept samples with different contents of the $R^4/S^5$ truncated variant for recombinant human Activin A was determined using the SPR technique (Biacore T200). The capture method was used for analysis. Protein A was first immobilized on the reference and experimental channels of the CM5 chip by amino coupling in a coupling amount of about 10,000RU. The test sample was captured on the experimental channel at a flow rate of 30 μl/min. Recombinant human Activin A protein at 2.5 nM was serially diluted two-fold with a running buffer to 0.078125 nM, and 2.5nM~0.078125nM serial concentrations of the recombinant human Activin A proteins were sequentially passed through the reference channel and the experimental channel, followed by binding for 3 min and dissociation for 20 min. After completion of each cycle, regeneration was performed twice using glycine-hydrochloric acid (pH 1.5) for 30 seconds each time. The obtained data were analyzed by Biacore T200 Evaluation software and fitted by 1:1 binding model.

Table 7 . Affinity of sotatercept samples with different contents of the $R^4/S^5$ truncated variant for Activin A

| Content of $R^4/S^5$ Truncated Variant (%) | ka (1/Ms) | kd (1/s) | $K_D$ (M) |
|---|---|---|---|
| 0.33 | 2.82E+07 | 1.76E-04 | 6.24E-12 |
| 3.85 | 3.51E+07 | 2.18E-04 | 6.21E-12 |
| 8.58 | 3.12E+07 | 2.24E-04 | 7.20E-12 |

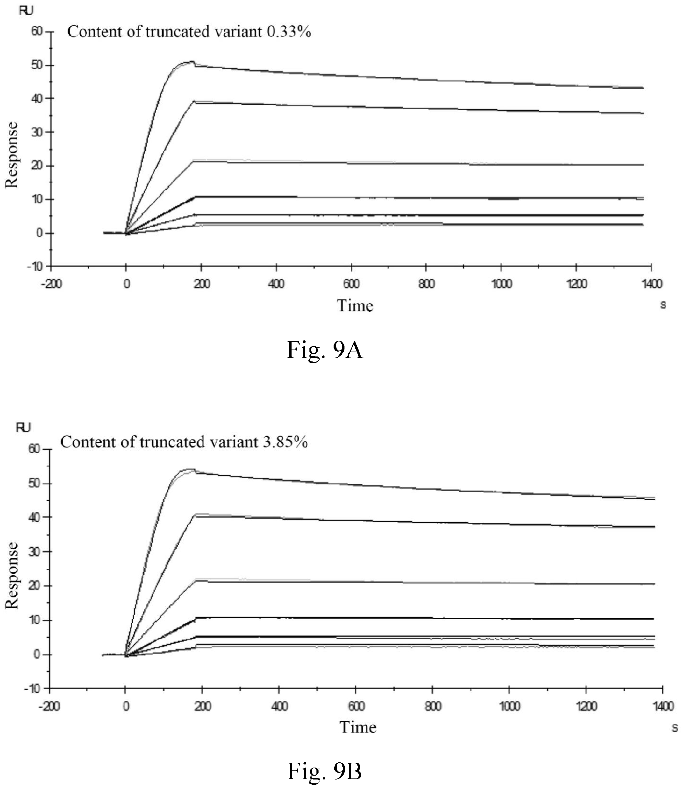

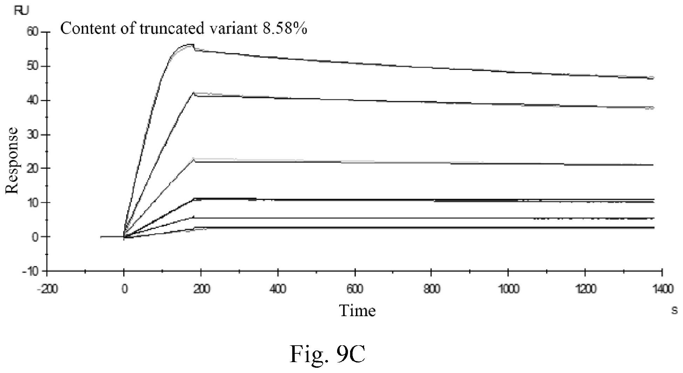

The analysis results of the affinity of the sotatercept samples with different contents of the $R^4/S^5$ truncated variant for Activin A were shown in Table 7, and the fitted graphs of binding-dissociation curves were shown in FIGS. 9A, 9B and 9C. The results showed that where the content of the $R^4/S^5$ truncated variant was within 10%, the affinity of the sotatercept samples with different contents of the $R^4/S^5$ truncated variant for Activin A was substantially consistent, and the fitted graphs of binding-dissociation curves were substantially consistent. Therefore, when the content of the $R^4/S^5$ truncated variant is less than or equal to about 10%, it will not affect the affinity and binding activity of the sotatercept samples for Activin A.

Various changes and equivalent substitutions can be made to the embodiments disclosed herein without departing from the spirit and scope of the disclosure. Unless otherwise indicated in the context, any feature, step, or embodiment of the disclosed embodiments can be used in combination with any other features or embodiments.

## Claims

**1.** A composition comprising sotatercept and a variant thereof, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2, and as calculated by a peptide mass fingerprinting analysis, the content of monomeric peptide chains with the peptide bond cleavage occurred between Arg(4) -Ser(5) is less than or equal to about 10% of the total amount of monomeric peptide chains in the composition.

**2.** The composition of claim 1, wherein as calculated by a peptide mass fingerprinting analysis, the content of monomeric peptide chains without the peptide bond cleavage occurred between Arg(4)-Ser(5) is greater than or equal to about 90% of the total amount of monomeric peptide chains in the composition.

**3.** The composition of claim 1 or 2, wherein the content of monomeric peptide chains with the peptide bond cleavage occurred between Arg(4) -Ser(5) is greater than or equal to about 0.1%, about 0.5%, about 1%, about 1.5%, or about 2% of the total amount of monomeric peptide chains in the composition.

**4.** The composition of claim 1 or 2, wherein the content of monomeric peptide chains with the peptide bond cleavage occurred between Arg(4) -Ser(5) is less than or equal to about 10%, about 9%, about 8%, about 7%, about 6%, about 5%, about 4%, about 3%, about 2.5%, about 2%, about 1.5%, about 1%, about 0.9%, 0.8%, about 0.7%, about 0.6%, or about 0.5% of the total amount of monomeric peptide chains in the composition.

**5.** The composition of claim 1 or 2, wherein the composition is prepared by a method comprising the steps of:

culturing host cells comprising a nucleic acid molecule encoding an amino acid sequence as set forth in SEQ ID NO:2 or a vector comprising the nucleic acid molecule in a bioreactor, wherein the culturing comprises a cell expansion phase and a protein production phase, and the culture temperature during the protein production phase is substantially maintained at 29.5°C-37°C; and/or
wherein the composition is prepared by a method comprising the steps of:
culturing host cells comprising a nucleic acid molecule encoding an amino acid sequence as set forth in SEQ ID NO:2 or a vector comprising the nucleic acid molecule in a bioreactor, wherein the average daily addition concentration of iron ions during the culture period is from 1.5 μmol/L/day to 125 μmol/L/day.

**6.** A method for preparing the composition of any one of claims 1-4, comprising culturing host cells comprising a nucleic acid molecule encoding an amino acid sequence as set forth in SEQ ID NO:2 or a vector comprising the nucleic acid molecule in a bioreactor, wherein the culturing comprises a cell expansion phase and a protein production phase, and the culture temperature during the protein production phase is substantially maintained at 29.5°C-37°C; and/or the culturing is performed using a culture medium supplemented with iron ions.

7. The method of claim 6, wherein the iron ions are derived from one or more of ferric citrate, ferric nitrate, ferrous sulfate, ferric ethylenediaminetetraacetate, and transferrin-bound iron.

8. The method of claim 7, wherein the iron ions are derived from ferric citrate; and/or

wherein the average daily addition concentration of the iron ions during the culture period is from 1.5 μmol/L/day to 125 μmol/L/day;
preferably, the average daily addition concentration of the iron ions is from 1.50 μmol/L/day to 5.00 μmol/L/day, 5.01 μmol/L/day to 15.00 μmol/L/day, 15.01 μmol/L/day to 30.00 μmol/L/day, 30.01 μmol/L/day to 45.00 μmol/L/day, 45.01 μmol/L/day to 60.00 μmol/L/day, 60.01 μmol/L/day to 75.00 μmol/L/day, 75.01 μmol/L/day to 90.00 μmol/L/day, 90.01 μmol/L/day to 115.00μmol/L/day, or 115.01 μmol/L/day to 125.00 μmol/L/day;
more preferably, the average daily addition concentration of the iron ions is selected from 1.77 μmol/L/day, 7.88 μmol/L/day, 62.85 μmol/L/day, 93.39 μmol/L/day, or 123.93 μmol/L/day.

9. The method of claim 6, wherein the culture temperature is maintained at least 29.5°C, at least 30°C, at least 30.5°C, at least 31°C, at least 31.5°C, at least 32°C, at least 32.5°C, at least 33°C, at least 33.5°C, at least 34°C, at least 34.5°C, at least 35°C, at least 35.5°C, at least 36°C, at least 36.5°C, or at least 37°C.

10. The method of claim 6, wherein the culture temperature is maintained at 29.5°C-37°C for at least 50% to 100% of the time during the protein production phase;

preferably, the culture temperature is maintained at 29.5°C-37°C for at least 50%, at least 55%, at least 60%, at least 65%, at least 70%, at least 75%, at least 80%, at least 85%, at least 90%, at least 95%, at least 98% or at least 99% of the time during the protein production phase;
more preferably, the culture temperature is maintained at 29.5°C-37°C for substantially 95% of the time during the protein production phase.

11. The method of any one of claims 6-10, wherein the host cells are mammalian cells;

preferably, the mammalian cells are derived from a murine or a human;
more preferably, the cells derived from the human include HEK 293 or PER.C6; and/or the cells derived from the murine include CHO, Hybridoma, YB2/0, NS0, Sp2/0 or BHK;
most preferably, the host cells are CHO cells.

12. A pharmaceutical formulation comprising the composition of any one of claims 1-5 or the composition prepared according to the method of any one of claims 6-11, and one or more pharmaceutically acceptable excipients.

13. The composition of any one of claims 1-5, the composition prepared according to the method of any one of claims 6-11 or the pharmaceutical formulation of claim 12, for use in treating pulmonary arterial hypertension or skeletal diseases.

14. Use of a culture medium comprising iron ions in reducing the content of a sotatercept variant in a sotatercept-containing product, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2; preferably, the iron ions are derived from ferric citrate.

15. A method for detecting a sotatercept variant in a sotatercept-containing product, wherein the variant has a peptide bond cleavage occurred between arginine at position 4 and serine at position 5 (Arg(4)-Ser(5)) in the amino acid sequence of one or two monomeric peptide chains of sotatercept as set forth in SEQ ID NO:2, and wherein the method comprises:

subjecting the product to lysyl endonuclease (Lys-C) digestion to obtain the digestion product; and
subjecting the digestion product to a peptide mass fingerprinting analysis by using LC-MS/MS, and determining the presence or absence of the variant based on MS1 and MS2 spectra;
optionally, the method further comprises obtaining XIC chromatograms of the truncated peptide segment and the full-length peptide segment after performing a peptide mass fingerprinting analysis when the variant is determined to be present, and calculating the truncated variant content from the corresponding peak areas.

R/S cleavage sites
Extracellular region of activin IIA receptor
Human IgG1 Fc region

Fig. 1

B
77630.0
100
%
75
50
25
A
77191.0
77792.0
77995.0
77000
77500
78000
78500
Mass [Da]
R/S cleavage sites
Extracellular region of activin IIA receptor
Human IgG1 Fc region

Fig. 2

36.33
Relative abundance
100
80
60
40
20
0
26
28
30
32
34
36
38
40
42
44
46
48
50
Time(minutes)

Fig. 3A

951.9207
952.4217
952.9225
953.4234
953.9242
Relative abundance
100
80
60
40
20
0
950
951
952
953
954
955
956
957
958
Mass-to-charge ratio

Fig. 3B

Relative abundance
Mass-to-charge ratio
y1 147.11
b2 217.08
y2 276.16
359.16
b3
y3 462.23
b4
557.22
y4 576.28
y5 647.32
y6 761.36
b6
784.36
b7
y7 908.43
b8
y8 1055.49
y9 1168.58
y10 1328.61
y11 1457.65
b12

Fig. 3C

| Source | LogWorth | | p value |
|---|---|---|---|
| Temperature (32,36.5) | 5.653 | | 0.00000 |
| Ferric citrate (62.85,123.93) | 2.378 | | 0.00418 |
| Riboflavin (0.082,0.609) | 0.672 | | 0.21289 |
| Lithium chloride (0,3.523) | 0.173 | | 0.67129 |
| Ammonium metavanadate (0.00026,0.901) | 0.033 | | 0.92605 |

Fig. 4

RS cleavage
1.0644
[-0.1027,
2.23153]
0.082
Riboflavin
62.85
Ferric citrate
0.00026
Ammonium metavanadate
0
Lithium chloride
32
Temperature

Fig. 5

Content change of the R4/S5 truncated variant under different culture temperatures and average daily addition concentrations of ferric citrate
Content of the R4/S5 truncated variant(%)
6
5
4
3
2
1
0
62.85
93.39
123.93
62.85
93.39
123.93
62.85
93.39
123.93
36.5°C±0.5°C
34°C±0.5°C
32°C±0.5°C
Culture temperatures and average daily addition concentrations of ferric citrate(μmol/L day)

Fig. 6

Content change of the R4/S5 truncated variant at different incubation temperatures
Content of the R4/S5 truncated variant(%)
6
5
4
3
2
1
0
36.5°C±0.5°C
34°C±0.5°C
32°C±0.5°C
30°C±0.5°C
Culture temperatures(°C)

Fig. 7

Content change of the R4/S5 truncated variant under different average daily addition concentrations of ferric citrate
Content of the R4/S5 truncated variant(%)
10
9
8
7
6
5
4
3
2
1
0
1.77
7.88
62.85
93.39
123.93
Average daily addition concentrationsof ferric citrate(μmol/L day)

Fig. 8

RU
Content of truncated variant 0.33%
Response
60
50
40
30
20
10
0
-10
-200
0
200
400
600
800
1000
1200
1400
Time
s

Fig. 9A

RU
Content of truncated variant 3.85%
Response
60
50
40
30
20
10
0
-10
-200
0
200
400
600
800
1000
1200
1400
Time
s

Fig. 9B

RU
Content of truncated variant 8.58%
60
50
40
30
20
10
0
-10
Response
-200
0
200
400
600
800
1000
1200
1400
Time
s

Fig. 9C

# EUROPEAN SEARCH REPORT

Application Number
EP 26 17 0116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/262718 A1 (ACCELERON PHARMA INC [US]) 30 December 2021 (2021-12-30) * example 1; sequence 27 * ----- | 1-15 | INV. C07K14/71 A61K38/00 A61P9/12 C12N5/071 |
| X | WO 2016/090077 A1 (CELGENE CORP [US]; ACCELERON PHARMA INC [US]) 9 June 2016 (2016-06-09) * paragraph [0187]; sequence 13 * ----- | 1-15 | |
| A | WO 2020/251924 A1 (REGENERON PHARMA [US]) 17 December 2020 (2020-12-17) * example 4; sequence 38 * ----- | 1-15 | |
| A | WO 2022/098570 A1 (HAN HQ [US]; ZHOU XIAOLAN [US]) 12 May 2022 (2022-05-12) * paragraphs [0221], [0224], [0226]; claim 1; sequence 55 * ----- | 1-15 | |
| A | WO 2021/189019 A1 (KEROS THERAPEUTICS INC [US]) 23 September 2021 (2021-09-23) * claim 1; example 1; tables 5,6; sequence 219 * ----- | 1-15 | TECHNICAL FIELDS SEARCHED (IPC) |
| A | US 8 637 312 B2 (KRUEGER OLAF [DE]; OELLERS KERSTIN [DE] ET AL.) 28 January 2014 (2014-01-28) * claim 1; figure 4; example 4 * ----- | 1-15 | C07K A61P C12N A61K |
| A | MIRANDA AIMON C. ET AL: "Sotatercept: A First-In-Class Activin Signaling Inhibitor for Pulmonary Arterial Hypertension", JOURNAL OF PHARMACY TECHNOLOGY, vol. 41, no. 3, 22 February 2025 (2025-02-22), pages 134-143, XP093408172, US ISSN: 8755-1225, DOI: 10.1177/87551225251317957 * abstract * ----- | 1-15 | |

The present search report has been drawn up for all claims

1

| Place of search | Date of completion of the search | Examiner |
|---|---|---|
| Munich | 19 June 2026 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 26 17 0116

This annex lists the patent family members relating to the patent documents cited in the above-mentioned European search report. The members are as contained in the European Patent Office EDP file on
The European Patent Office is in no way liable for these particulars which are merely given for the purpose of information.

19-06-2026

| Patent document cited in search report | Publication date | Patent family member(s) | Publication date |
|---|---|---|---|
| WO 2021262718 A1 | 30-12-2021 | AU 2021296421 A1 | 19-01-2023 |
| | | BR 112022025644 A2 | 07-03-2023 |
| | | CA 3182838 A1 | 30-12-2021 |
| | | CL 2022003686 A1 | 26-05-2023 |
| | | CN 115989035 A | 18-04-2023 |
| | | CO 2022018719 A2 | 07-03-2023 |
| | | EC SP23004827 A | 31-03-2023 |
| | | EP 4168037 A1 | 26-04-2023 |
| | | IL 299189 A | 01-02-2023 |
| | | JP 7854402 B2 | 01-05-2026 |
| | | JP 2023534143 A | 08-08-2023 |
| | | KR 20230026435 A | 24-02-2023 |
| | | PE 20230783 A1 | 11-05-2023 |
| | | US 2023226146 A1 | 20-07-2023 |
| | | US 2025082722 A1 | 13-03-2025 |
| | | US 2025249070 A1 | 07-08-2025 |
| | | WO 2021262718 A1 | 30-12-2021 |
| WO 2016090077 A1 | 09-06-2016 | AU 2015358469 A1 | 29-06-2017 |
| | | AU 2021212084 A1 | 26-08-2021 |
| | | BR 112017011722 A2 | 27-02-2018 |
| | | CA 2969572 A1 | 09-06-2016 |
| | | CN 107533040 A | 02-01-2018 |
| | | CN 114675039 A | 28-06-2022 |
| | | CY 1126033 T1 | 15-11-2023 |
| | | DK 3227675 T3 | 30-05-2023 |
| | | EP 3227675 A1 | 11-10-2017 |
| | | EP 4233889 A2 | 30-08-2023 |
| | | ES 2946160 T3 | 13-07-2023 |
| | | FI 3227675 T3 | 25-05-2023 |
| | | HK 1245397 A1 | 24-08-2018 |
| | | HK 1248807 A1 | 19-10-2018 |
| | | HR P20230504 T1 | 15-09-2023 |
| | | HU E062189 T2 | 28-09-2023 |
| | | JP 7092853 B2 | 28-06-2022 |
| | | JP 2018501307 A | 18-01-2018 |
| | | JP 2021038249 A | 11-03-2021 |
| | | KR 20170098851 A | 30-08-2017 |
| | | LT 3227675 T | 12-06-2023 |
| | | MD 20180012 A2 | 31-05-2018 |
| | | NZ 732343 A | 29-11-2024 |
| | | NZ 770419 A | 29-11-2024 |
| | | PL 3227675 T3 | 17-07-2023 |
| | | PT 3227675 T | 30-05-2023 |
| | | RS 64214 B1 | 30-06-2023 |
| | | SI 3227675 T1 | 31-07-2023 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 26 17 0116

This annex lists the patent family members relating to the patent documents cited in the above-mentioned European search report. The members are as contained in the European Patent Office EDP file on
The European Patent Office is in no way liable for these particulars which are merely given for the purpose of information.

19-06-2026

| Patent document cited in search report | Publication date | Patent family member(s) | Publication date |
|---|---|---|---|
| | | SM T202300166 T1 | 20-07-2023 |
| | | TW 201636044 A | 16-10-2016 |
| | | TW 202123961 A | 01-07-2021 |
| | | US 2017360887 A1 | 21-12-2017 |
| | | WO 2016090077 A1 | 09-06-2016 |
| WO 2020251924 A1 | 17-12-2020 | AU 2020292246 A1 | 27-01-2022 |
| | | CA 3141266 A1 | 17-12-2020 |
| | | EP 3983436 A1 | 20-04-2022 |
| | | JP 7807238 B2 | 27-01-2026 |
| | | JP 2022537142 A | 24-08-2022 |
| | | JP 2025138816 A | 25-09-2025 |
| | | MA 56203 A | 20-04-2022 |
| | | US 2022251184 A1 | 11-08-2022 |
| | | WO 2020251924 A1 | 17-12-2020 |
| WO 2022098570 A1 | 12-05-2022 | AU 2021376241 A1 | 22-06-2023 |
| | | CA 3196443 A1 | 12-05-2022 |
| | | EP 4240388 A1 | 13-09-2023 |
| | | JP 2023548399 A | 16-11-2023 |
| | | KR 20230117348 A | 08-08-2023 |
| | | US 2024343788 A1 | 17-10-2024 |
| | | WO 2022098570 A1 | 12-05-2022 |
| WO 2021189019 A1 | 23-09-2021 | AU 2021237730 A1 | 06-10-2022 |
| | | CA 3176735 A1 | 23-09-2021 |
| | | CN 115768457 A | 07-03-2023 |
| | | EP 4121078 A1 | 25-01-2023 |
| | | IL 296394 A | 01-11-2022 |
| | | JP 2023518260 A | 28-04-2023 |
| | | JP 2025179059 A | 09-12-2025 |
| | | KR 20230004515 A | 06-01-2023 |
| | | US 2023079602 A1 | 16-03-2023 |
| | | US 20260098078 A1 | 09-04-2026 |
| | | WO 2021189019 A1 | 23-09-2021 |
| US 8637312 B2 | 28-01-2014 | CA 2707524 A1 | 16-07-2009 |
| | | CN 102317440 A | 11-01-2012 |
| | | DK 2250251 T3 | 22-01-2018 |
| | | EP 2250251 A1 | 17-11-2010 |
| | | IL 206900 A | 31-10-2017 |
| | | JP 5431361 B2 | 05-03-2014 |
| | | JP 2011509089 A | 24-03-2011 |
| | | KR 20100108424 A | 06-10-2010 |
| | | US 2010285533 A1 | 11-11-2010 |
| | | WO 2009087087 A1 | 16-07-2009 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 26 17 0116

This annex lists the patent family members relating to the patent documents cited in the above-mentioned European search report. The members are as contained in the European Patent Office EDP file on
The European Patent Office is in no way liable for these particulars which are merely given for the purpose of information.

19-06-2026

| Patent document cited in search report | Publication date | Patent family member(s) | Publication date |
| --- | --- | --- | --- |
| ------------------------------------------------------------------ | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202511424337X

### Non-patent literature cited in the description

- *CHEMICAL ABSTRACTS*, 1001080-50-7